# EUROPEAN PATENT APPLICATION

(11) **EP 3 466 330 A1**
(43) Date of publication of application: **10.04.2019**
(21) Application number: 17803128.2
(22) Date of filing: 29.05.2017
(51) Int. Cl.: A61B 5/04, A61B 5/00

(54) **BIO-IMPLANTABLE DEVICE AND METHOD FOR MANUFACTURING SAME**

(30) Priority: 27.05.2016 KR 20160065292
(71) Applicant: Todoc Co., Ltd., Guro-gu, Seoul 08394 (KR)
(72) Inventor: MIN, Kyou-Sik, Suwon-si Gyeonggi-do 16493 (KR)
(74) Representative: Stöckeler, Ferdinand
(86) International application number: PCT/KR2017/005598
(87) International publication number: WO 2017/204610

(57) **Abstract**

The present disclosure provides a bio-implantable device and a method for manufacturing the same, the bio-implantable device comprising: a substrate section made of a polymer; a wi re section formed on a surface of the substrate section; at le ast one micro wire array comprising a cover section made of a polymer and attached to the surface of the substrate section, on which the wire section is formed, so as to protect the wire section and to expose at least one end of the wire section, t hereby forming a pad section; a functioning section electrica lly connected to the pad section; and at least one package com prising an encapsulating section made of a polymer so as to ab ut the functioning section and the pad section with no space t herebetween, thereby protecting the same, the encapsulating s ection extending up to a part adjacent to the substrate sectio n and to the pad section of the cover section and being attach ed thereto, the package being connected to an end of a corresp onding micro wire array.

## Description

### Technical Field

The present disclosure relates generally to a bio-implantable device and, more particularly, to a bio-implantable device using a polymer and a method of manufacturing the same.

### Background Art

Bio-adhesive or bio-implantable structures for collecting biochemical responses and bio-signals by conversion into electrical signals and delivering electrical signals for neural stimulation to neural tissues are
implantable devices respectively including a microelectrode array and a package are also referred to as microelectrode array packages.

In a bio-implantable device, such as a bio-implantable stimulator, a bio-implantable detector, a cardiac pacemaker, a neural prosthesis, or a neuromodulator, the encapsulated state thereof is critical, since the bio-implantable device operates, with at least a portion thereof being implanted or inserted into a living body. When the encapsulated state of the device is inadequate, a body fluid may leak into an electronic circuit present within the device, thereby causing a variety of problems, such as a breakdown, malfunction, and shortened longevity, to the device.

Korean Patent No. 10-1088806 (corresponding US Patent No. 8,886,277 B2) describes a related-art approach in which a package and microelectrodes are connected using feed-throughs, and indicates that a bio-implantable device of the related-art approach disadvantageously has low encapsulation property due to heterogeneous binding.

In addition, although there is another related-art approach of forming a package and microelectrodes using a liquid crystal polymer (LCP) in order to overcome the disadvantageous low encapsulation property due to heterogeneous binding, this related-art approach is also indicated as having a problem. The above-stated document discloses a microelectrode array package using a liquid polymer and a method of manufacturing the same, in which the microelectrode array package is more reliable, and the processing period thereof is reduced.

However, a technical solution able to improve the endurance and reliability of a bio-implantable using a polymer and improve the convenience of a manufacturing process is still demanded.

### Disclosure

### Technical Problem

Accordingly, the present disclosure has been made keeping in mind the above problems occurring in the prior art, and an object of the present disclosure is to provide a bio-implantable device and a method of manufacturing the same, the device and method having superior endurance and reliability and excellent convenience in a manufacturing process.

### Technical Solution

A bio-implantable device according to the present disclosure includes one or more microwire arrays and one or more packages connected to one end of a corresponding microwire array among the microwire arrays. Each of the one or more microwire arrays includes: a substrate section made of a polymer; a wire section provided on one surface of the substrate section; and a cover section made of a polymer, the cover section being adhered to the one surface of the substrate section on which the wire section is provided to protect the wire section and expose at least one end of the wire section, thereby providing a pad section. Each of the one or more packages includes: a functioning section electrically connected to the pad section via a terminal section; and an encapsulating section made of a polymer, the encapsulating section being in contact with the functioning section and the pad section without gaps to protect the functioning section and the pad section, and extending to and being adhered to portions of the substrate section and the cover section adjacent to the pad section. The encapsulating section is provided by curing of the polymer, an initial state of which is liquid.

A method of manufacturing a bio-implantable device according to the present disclosure includes: (a) a step of preparing one or more microwire arrays respectively including a wire section disposed between a substrate section and a cover section made of polymers, with a portion of the wire section being exposed from the cover section to provide a pad section on at least one end thereof; (b) a step of electrically connecting the one or more microwire arrays and one or more functioning sections by electrically connecting terminal sections of the one or more functioning sections to the pad sections of the one or more microwire arrays; and (c) a step of providing an encapsulating section in contact with the one or more functioning sections and the pad sections without gaps to protect the one or more functioning sections and the pad sections by placing the electrically-connected one or more microwire arrays and functioning sections in a mold, injecting a liquid polymer into the mold, and cooling the injected polymer, the encapsulating section extending and being adhered to portions of the substrate sections and the cover sections adjacent to the pad sections.

### Advantageous Effects

The bio-implantable device according to the present disclosure has superior endurance and reliability, as well as excellent convenience in a manufacturing process.

### Description of Drawings

FIG. 1 is a longitudinal cross-sectional view illustrating a bio-implantable device according to an embodiment of the present disclosure;
FIG. 2 is a plan view illustrating the bio-implantable device according to an embodiment of the present disclosure;
FIG. 3 is a plan view illustrating a bio-implantable device according to another embodiment of the present disclosure;
FIGS. 4, 5, and 6 are views sequentially illustrating processes of a method of manufacturing the bio-implantable device according to an embodiment of the present disclosure; and
FIG. 7 is a view sequentially illustrating a process of constructing the functioning section in a method of manufacturing the bio-implantable device according to another embodiment of the present disclosure.

### Best Mode

Advantages and features of the present disclosure, as well as implementation methods thereof, will be clarified through following embodiments described with reference to the accompanying drawings. The present invention may, however, be embodied in different forms and should not be construed as limited to the embodiments set forth herein. Rather, these embodiments are provided so that this disclosure will be thorough and complete, and will fully convey the scope of the present invention to those skilled in the art. Further, the present invention is only defined by scopes of claims. The terms used herein shall be interpreted as being illustrative of the following embodiments, while not being limitative of the present invention. In addition, the terms of a singular form used herein may include plural forms unless referred to the contrary.

It should be understood that, throughout different drawings, the same reference numerals and symbols will be used to designate the same or like components. In the following description of the embodiments, detailed descriptions of known functions and components incorporated herein will be omitted in the case that the subject matter of the present disclosure may be rendered unclear thereby.

It should be understood that the terms, such as "one end", "the other end", and "end", used herein do not necessarily indicate a most distal end but indicate a portion more adjacent to a distal portion rather than to a central portion.

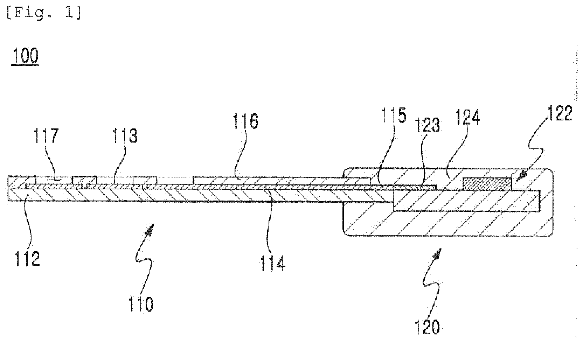

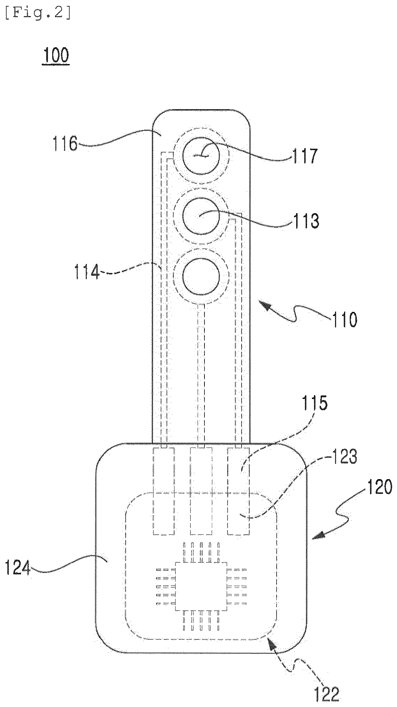

FIG. 1 is a longitudinal cross-sectional view illustrating a bio-implantable device according to an embodiment of the present disclosure, and FIG. 2 is a plan view illustrating the bio-implantable device according to an embodiment of the present disclosure.

As illustrated in FIGS. 1 and 2, a bio-implantable device 100 according to the present disclosure includes a microwire array 110 and a package 120. The microwire array 110 includes a substrate section 112 made of a polymer, a wire section 114 provided on one surface of the substrate section 112, and a cover section 116 made of a polymer and cover sectioning and protecting the wire section 114. When the bio-implantable device 100 operates, signals, current, electric power, and the like are transmitted through the wire section 114 of the microwire array 110.

The cover section 116 cover sections and protects the wire section 114, such that at least one end of the wire section 114 is exposed from the cover section 116 to provide a pad section 115. FIGS. 1 and 2 illustrate one end of the wire section 114 being exposed to provide the pad section 115. According to the present embodiment, the cover section 116 is provided with electrode holes 117 in the other end thereof, opposite to one end thereof on which the pad section 115 is provided, to expose an electrode section 113. When the bio-implantable device 100 collects a bio-signal, the electrode section 113 functions as inlets for the bio-signal. When the bio-implantable device 100 emits a bio-stimulation, the electrode section 113 functions as outlets for the bio-stimulation signal. The microwire array 110 provided with the electrode section 113 may also be referred to as a "microelectrode array."

The microwire array 110 is coupled to the package 120. The package 120 includes a functioning section 122 and an encapsulating section 124. The functioning section 122 performs a variety of functions necessary for the bio-implantable device 100 to perform ordinary operations, such as signal processing, communication, and the like, and is typically comprised of one or more functional modules. The functioning section 122 is provided with a terminal section 123 electrically connected to the pad section 115 of the microwire array 110, such that signals, current, electric power, and the like may flow to the pad section 115 through the terminal section 123 and vice versa.

The functioning section 122 and pad section 115 are protected from the external environment by the encapsulating section 124 made of a polymer. The encapsulating section 124 is in contact with the functioning section 122 and the pad section 115 without gaps therebetween to protect the functioning section 122 and the pad section 115. In addition, the encapsulating section 124 extends to reach and is adhered to portions of the substrate section 112 and the cover section 116. That is, the encapsulating section 124 extends and is adhered to the portions of the substrate section 112 and the cover section 116 adjacent to the pad section 115.

Since the encapsulating section 124 is made of a polymer, i.e. the same material as those of the substrate section 112 and the cover section 116, the problem of low encapsulation property due to heterogeneous binding does not occur after the encapsulating section 124 is adhered to the substrate section 112 and the cover section 116. Since the encapsulating section 124 is in contact with and supports the functioning section 122 and the pad section 115 without gaps therebetween, the endurance and reliability of the bio-implantable device 100 are improved.

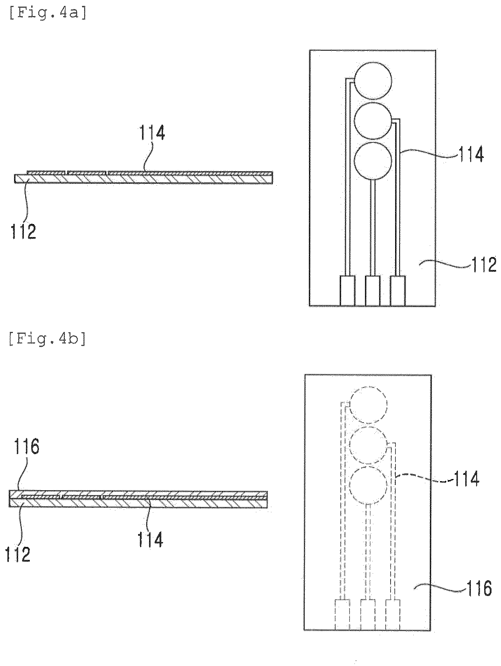

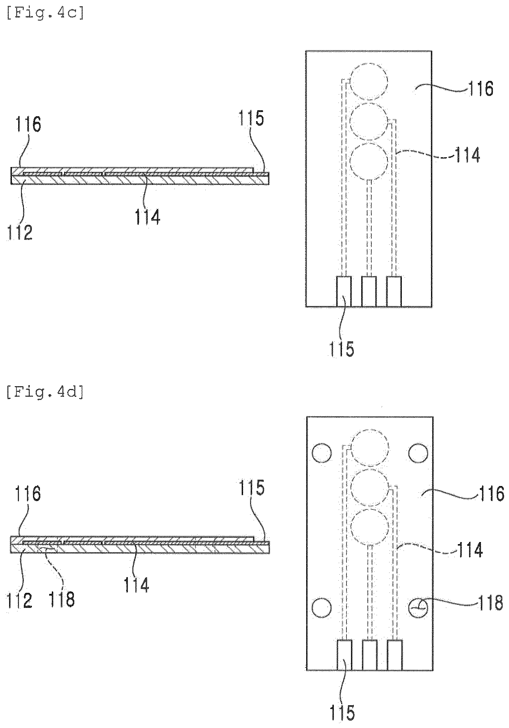

The encapsulating section 124 is formed by curing of the polymer, the initial state of which is liquid. The term "initial state" used herein refers to a state at a point in time at which the encapsulating section 124 initially occupied the space that the encapsulating section 124 is occupying. This means that the polymer has occupied the space of the encapsulating section 124 from the initial stage, in which the polymer is liquid instead of being, for example, powder or a film, and the liquid polymer is cured to form the encapsulating section 124. When the initial state is powder or a film, the powder or film must be subjected to, for example, high-temperature and high-pressure processing for 30 minutes or more to form the encapsulating section 124. Consequently, the functioning section 122 is subjected to a high-pressure and high-temperature atmosphere for a long time, thereby causing a breakdown in an electronic component of the functioning section 122. However, in the case in which the encapsulating section 124 is formed by curing the polymer having the liquid initial state, for example, when the method illustrated in FIGS. 4 to 7 is used, it is enough to apply the high-pressure and high-temperature atmosphere for about 3 to 5 seconds only to form the encapsulating section 124. Accordingly, a disadvantageous breakdown in an electronic component of the functioning section 122 does not occur.

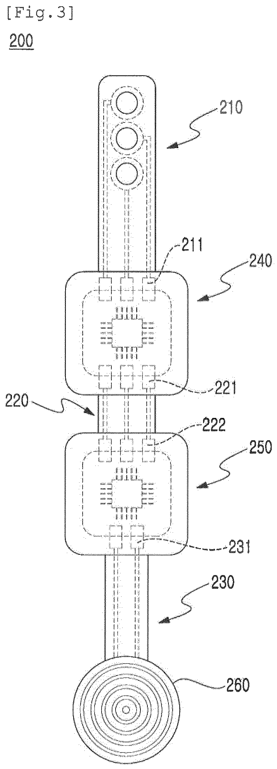

FIG. 3 is a plan view illustrating a bio-implantable device according to another embodiment of the present disclosure.

As illustrated in FIG. 3, a bio-implantable device 200 may include two or more wire arrays 210, 220, and 230 and two or more packages 240 and 250. The configuration of each of the microwire arrays 210, 220, and 230 is the same as the configuration of the above-described microwire array 110, and the configuration of each of the packages 240 and 250 is the same as the configuration of the above-described package 120. The first microwire array 210 is provided with a pad section 211 on one end thereof, since the one end is connected to the first package 240. The second microwire array 220 is provided with pad sections 221 and 222 on both ends thereof, since one end thereof is connected to the first package 240 and the other end thereof is connected to the second package 250. The third microwire array 230 is provided with a pad section 231 on one end thereof, since the one end is connected to the second package 250. Although the other end of the third microwire array 230 is not connected to a package, the other end is connected to a coil 260. For this purpose, the third microwire array 230 may also be provided with a pad section (not shown) on the other end. Although the coil 260 may be regarded a type of functioning section, the coil 260 is not provided as a package to which an encapsulating section is applied. Although this configuration may generally be applied to the functioning section present outside of a living body, the present disclosure is not limited to the functioning section present outside of a living body.

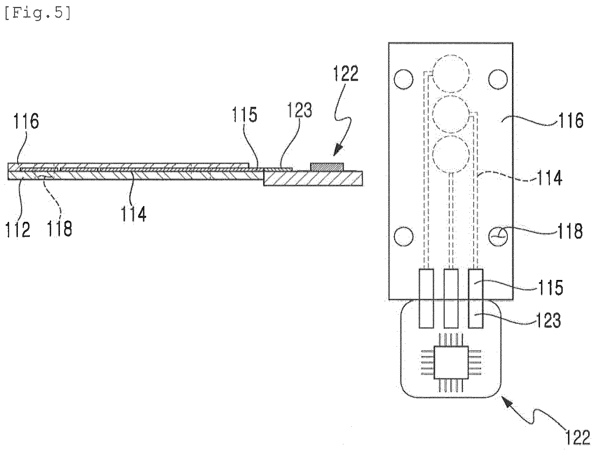

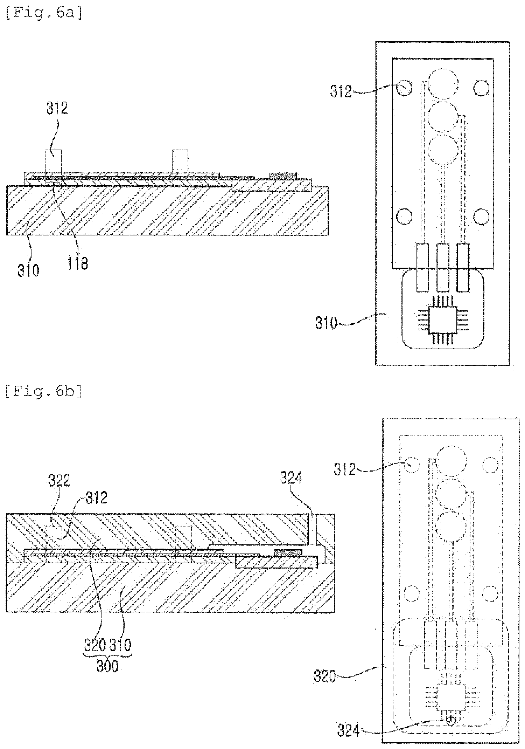

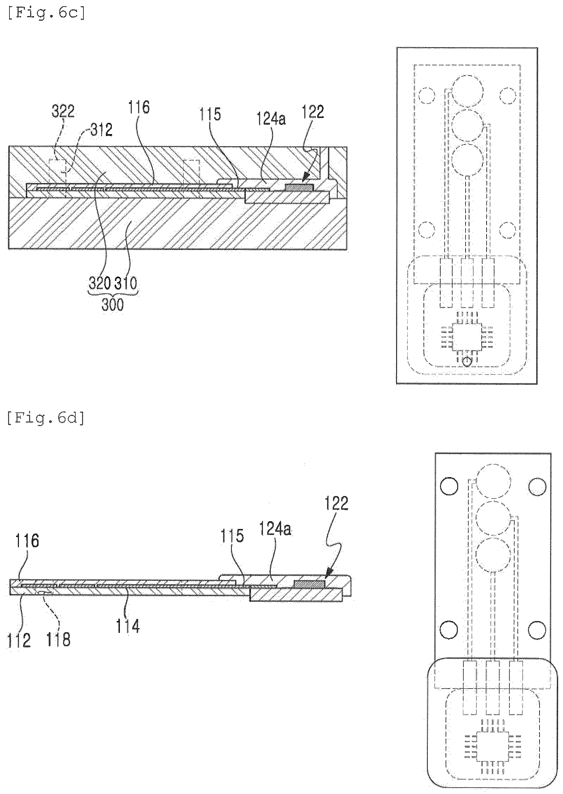

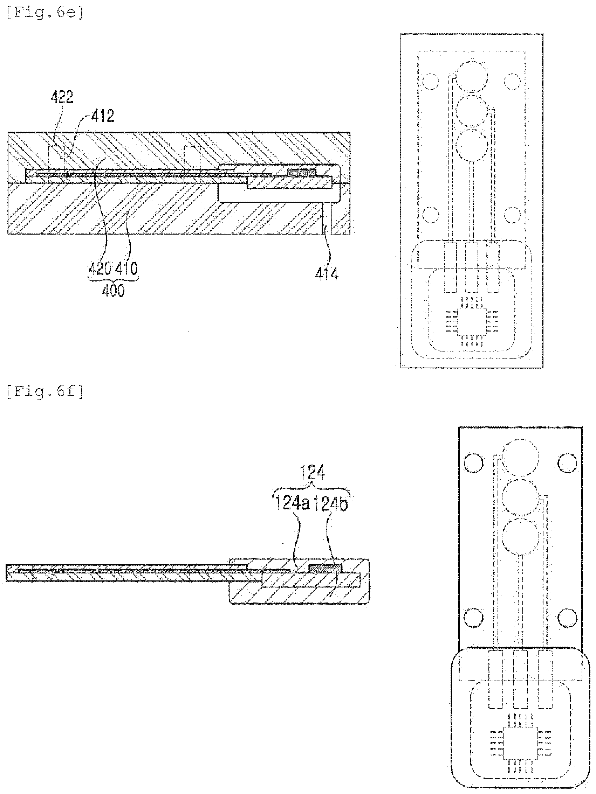

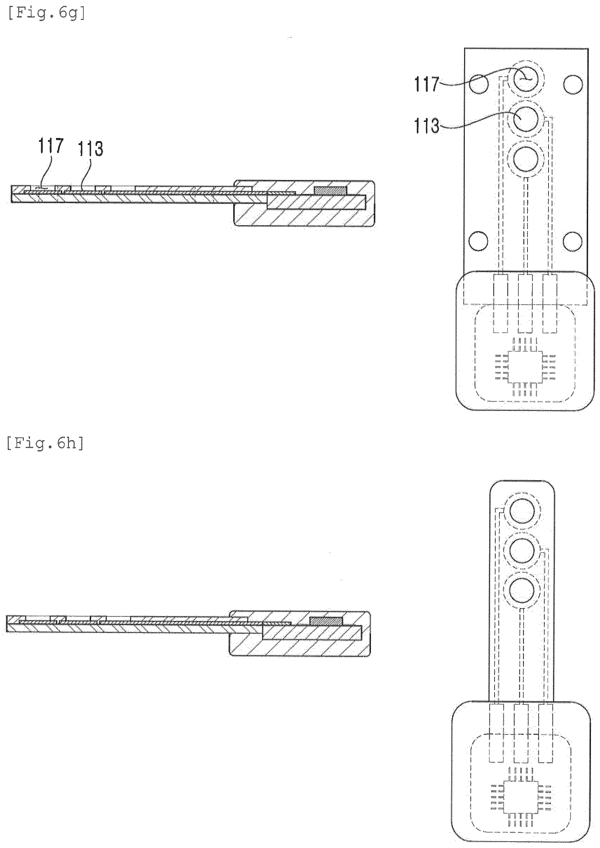

FIGS. 4, 5, and 6 are views sequentially illustrating processes of a method of manufacturing the bio-implantable device according to an embodiment of the present disclosure, in each of which a cross-sectional view is illustrated on the left, and a plan view is illustrated on the right. FIG. 5 illustrates a process subsequent to the process of FIG. 4, and FIG. 6 illustrates a process subsequent to the process of FIG. 5. Hereinafter, the processes will be described in the order in which the processes are illustrated in the drawings.

As illustrated in FIG. 4a, a step of forming the wire section 114 on one surface of the substrate section 112 made of a polymer is performed. The forming of the wire section 114 on one surface of the substrate section 112 may be performed by various known polymer-based microcircuit patterning methods, such as semiconductor processing. However, the present disclosure is not limited to a specific method.

As illustrated in FIG. 4b, a step of attaching the cover section 116 made of a polymer to the one surface of the substrate section 112, on which the wire section 114 is provided, is performed. The step of attaching the cover section 116 may be performed various known methods, such as thermocompression. However, the present disclosure is not limited to a specific method.

As illustrated in FIG. 4c, one end of the cover section 116 is processed so that a portion of the wire section 114 is exposed, thereby forming the pad section 115. The processing of the cover section 116 may be performed by various known methods, such as laser engraving (or etching). However, the present disclosure is not limited to a specific method.

As illustrated in FIG. 4d, alignment holes 118 are formed in regions, mainly in peripheral portions, of the substrate section 112 and the cover section 116, in which the wire section 114 is not present, so as to penetrate the substrate section 112 and the cover section 116. The order in which the alignment holes 118 and the pad section 115 are formed may be reversed, and the forming of the alignment holes 118 and the forming of the pad section 115 may be performed simultaneously. The alignment holes 118 are intended to be engaged with alignment pins of a mold for position alignment in a later process of being taken into the mold. In a case in which an alternative for position alignment is present, the forming of the alignment holes 118 may not be performed.

As described above, a single microwire array 100 is completed, and a larger number of microwire arrays 100 is manufactured as required.

However, the processes and the sequence thereof illustrated in FIG. 4 are illustrative only. The wire section 114 may be disposed between the substrate section 112 and the cover section 116, both of which are made of polymers. A step of providing the microwire array comprised of the wire section 114 and the pad section 115 is possible, with a portion of the wire section 114 forming the pad section 115 provided on at least one end and externally opened from the cover section 116. For example, the cover section 116 may be adhered to the substrate section 114 after completion of the processing on the cover section 116 for the pad section 115.

Afterwards, as illustrated in FIG. 5, the microwire array 110 and the functioning section 122 are electrically connected. That is, the pad section 115 of the microwire array 110 is electrically connected to the functioning section 122 corresponding thereto. In this regard, the terminal section 123 previously formed on the functioning section 122 and the pad section 115 of the microwire array 110 may be bonded using silver epoxy or hot lead. However, any of known technologies for electrically connecting the microwire array 110 and the functioning section 122 may be used, and these technologies shall be understood to be included in the present disclosure.

Afterwards, as illustrated in FIG. 6a, the microwire array 110 and the functioning section 122, electrically connected to each other, are seated on a lower plate 310 of a first mode (300, see FIG. 6b). Here, alignment pins 312 provided on the lower plate 310 of the first mold 300 are inserted into the alignment holes 118 provided in the peripheral portions of the microwire array 110, so that positions of the microwire array 110 and the functioning section 120 connected to the microwire array 110 are aligned.

As illustrated in FIG. 6b, an upper plate 320 of the first mold 300 is connected to the lower plate 310. The upper plate of the first mold 300 is provided with accommodation holes 322 into which the alignment pins 312 are inserted, such that strong coupling between the upper plate 320 and the lower plate 310 can be maintained. In addition, the upper plate of the first mold 300 is provided with a polymer inlet 324, and a hollow space within the first mold 300 extending from the polymer inlet 324 is suitably configured to form an upper encapsulating section 124a, which will be described later.

As illustrated in FIG. 6c, a liquid polymer is injected into the first mold 300 through the polymer inlet 324. After the injected polymer is cooled, the resultant structure is removed from the mold, so that the upper encapsulating section 124a is formed, as illustrated in FIG. 6d. The temperature of the first mold 300 is set to be sufficiently high (e.g. 200°C or higher) but lower than the melting temperature of the injected polymer, such that the polymer can be cured over time. This is intended to prevent the polymer injected into the first mold 300 from being cured before the inner space of the first mold 300 is filled with the polymer without gaps. The polymer cured as described above forms the upper encapsulating section 124a. The upper encapsulating section 124a formed as described above is in contact with the upper portions of the functioning section 122 and the pad section 115 without gaps therebetween to protect the functioning section 122 and the pad section 115, and extends and is adhered to a portion of the cover section 116 adjacent to the pad section 115. When the melting temperature of the polymer of the upper encapsulating section 124a is higher than the melting temperature of the cover section 116, a portion of the cover section 116, in contact with the uncured upper encapsulating section 124a, is melted and then is cured together with the upper encapsulating section 124a. This consequently improves adhesion of the upper encapsulating section 124a to the cover section 116 and further improves encapsulation property.

As illustrated in FIG. 6e, a second mold 400 comprised of a lower plate 410 and an upper plate 420 is used similarly to the first mold 300 in order to provide a second encapsulating section 124b (see FIG. 6f) in a location opposite to the first encapsulating section 124a (see FIG. 6f). Although the second mold 400 is illustrated that a polymer inlet 414 is provided in the lower plate 410, it will be clearly understood that the polymer inlet 414 is a component corresponding to the polymer inlet 324 provided in the upper plate 320 of the first mold 300 when turned upside down. In the drawing, reference numeral 412 indicates alignment pins provided in the lower plate 410, and 422 indicates accommodation holes provided in the upper plate 420 to accommodate the alignment pins 412 when the lower plate 410 and the upper plate 420 are connected. A liquid polymer is injected into the first mold 300 through the polymer inlet 414. After the injected polymer is cooled, the resultant structure is removed from the mold, so that the second encapsulating section 124b is formed in a location opposite to the upper encapsulating section 124a, as illustrated in FIG. 6f. The temperature of the second mold 400 is set to be sufficiently high (e.g. 200°C or higher) but lower than the melting temperature of the injected polymer, such that the polymer can be cured over time. This is intended to prevent the polymer injected into the second mold 400 from being cured before the inner space of the second mold 400 is filled with the polymer without gaps. The polymer cured as described above forms the lower encapsulating section 124b. The lower encapsulating section 124b formed as described above is in contact with lower portions of the functioning section 122 and the pad section 115 without gaps therebetween to protect the functioning section 122 and the pad section 115, and extends and is adhered to a portion of the substrate section 112 adjacent to the pad section 115. When the melting temperature of the polymer of the lower encapsulating section 124b is higher than the melting temperature of the substrate section 112, a portion of the substrate section 112, in contact with the uncured lower encapsulating section 124b, is melted and then is cured together with the lower encapsulating section 124b. This consequently improves adhesion of the lower encapsulating section 124b to the substrate section 112 and further improves encapsulation property.

According to the present embodiment, the first mold 300 and the second mold 400 are configured such that the upper encapsulating section 124a is formed first before the lower encapsulating section 124b is formed. However, in contrast, the molds may be configured such that the lower encapsulating section 124b is formed first before the upper encapsulating section 124a is formed. It should be understood that this configuration shall be within the scope of the present disclosure, and the scope of this configuration shall be defined by at least one claim of the Claims appended herein and all of its equivalents.

According to the present embodiment, the upper encapsulating section 124a and the lower encapsulating section 124b are formed using the separate molds 300 and 400 with a time difference. However, for example, forming both the upper encapsulating section 124a and the lower encapsulating section 124b using a single mold is within the scope of the present disclosure. That is, any process of forming the encapsulating section 124, which is in contact with the functioning section 122 and the pad section 115 without gaps therebetween to protect the functioning section 122 and the pad section 115 and extends and is adhered to portions of the substrate section 112 and the cover section 116 adjacent to the pad section 115, by placing the microwire array 110 and the functioning section 120 within the mold, injecting a liquid polymer into the mold, and cooling the injected polymer, is included within the scope of the present disclosure and the appended Claims.

Afterwards, as illustrated in FIG. 6g, the electrode section 113 is formed by processing the electrode holes 117 by a known method, such as laser engraving.

Afterwards, as illustrated in FIG. 6h, peripheral portions of the microwire array 110 are cut by any known method, such as laser cutting, thereby completing the bio-implantable device 100.

The embodiment illustrated in FIGS. 4 to 6 is the method of manufacturing the bio-implantable device 100 comprised of a single microwire array 110 and a single package 120, illustrated in FIS. 1 and 2. Manufacturing of the bio-implantable device 200 comprised of one or more microwire arrays 210, 220, and 230 and one or more microwire arrays 210, 220, and 230 may generally be performed by two methods.

According to the first method, after electrical connections for the bio-implantable device 200 are completed, a half-finished product, the electrical connections of which are completed, is disposed in a mold having an inner space configuration in which a plurality of packages spaced apart from each other can be formed. A liquid polymer for forming an encapsulating section is injected into the mold, is cooled, and removed from the mold. Accordingly, a plurality of packages is manufactured in a single time.

According to the second method, after electrical connections to elements designated with, for example, reference numerals 21, 240, and 220 in FIG. 3 are completed, the elements is disposed in a mold having a suitable shape, and a liquid polymer is injected into the mold and then is cooled. Due to these processes, a bio-implantable half-finished product comprised of the elements 210, 240, and 220 is completed. Afterwards, elements designated with reference numerals 250, 230, and 260 are additionally electrically connected to an element designated with reference numeral 220, and then the connected structure is disposed in a mold having a suitable shape. Subsequently, through processes of injecting a liquid polymer into the mold and cooling the polymer, the bio-implantable device 200, as illustrated in FIG. 3, is completed.

That is, in the latter case, a step of additionally forming one or more encapsulating sections is present. Specifically, in a state in which one or more encapsulating sections are formed, one or more functioning sections are added via microwire arrays, and the resultant structures are disposed in the mold. A liquid polymer is injected into the mold and then is cooled, thereby forming the one or more encapsulating sections. The encapsulating sections are in contact with the added functioning sections and pad sections connected to the functioning sections without gaps to protect the functioning sections and the pad sections, and extend and are adhered to portions of the substrate sections and the cover sections of the mediating microwire arrays adjacent to the pad sections. According to this method, bio-implantable device components and modules used for construction of a variety of bio-implantable devices may be previously manufactured, and the encapsulating section-forming method using the mold according to the present disclosure may be applied to connect the modules and components, thereby improving productivity and workability.

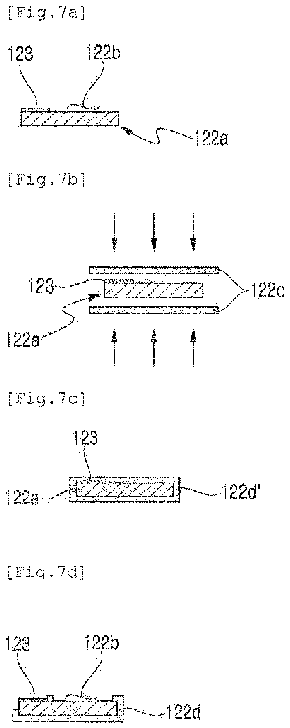

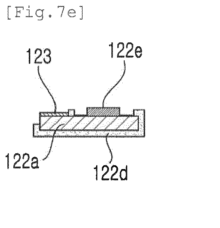

FIG. 7 is a view sequentially illustrating a process of constructing the functioning section in a method of manufacturing the bio-implantable device according to another embodiment of the present disclosure. Even in the case that the functioning section is used, the method of manufacturing a bio-implantable device is the same as the above-described method, except that steps of preparing the functioning section according the present embodiment are added. That is, it may be different that the functioning section illustrated in FIG. 7e is used in the step illustrated in FIG. 5.

Although the functioning section 122 generally includes a circuit board 122a and electronic components 122e (see FIG. 7e) mounted on the circuit board 122a, the embodiment illustrated in FIG. 7 additionally includes an adhesion-improving layer 122d (see FIGS. 7d and 7e). The bonding force-improving layer 122d is made of a polymer, the melting temperature of which is lower than the melting temperature of the polymer of the encapsulating section 124. As illustrated in FIGS. 7d and 7e, the adhesion-improving layer 122d surrounds portions of the circuit board 122a, except for a mounting section 122b allowing an electronic component 122e to be mounted thereon and a terminal section 123 electrically connected to the pad section 115 (see FIG. 5) .

The method of manufacturing the functioning section 122 according to the present embodiment will be described with reference to FIG. 7.

A circuit board 122a, as illustrated in FIG. 7a, is prepared. The circuit board 122a is provided with the terminal section 123 configured to be electrically connected to the pad section 115 (see FIG. 5) of the microwire array 110 and the mounting section 122b allowing an electric component 122e (see FIG. 7e) to be mounted thereon.

Polymer films 122c are laminated on the top surface and the bottom surface of the circuit board 122a, as illustrated in FIG. 7b, such that the substrate section 122a is wrapped in the polymer films 122c, thereby forming an intermediate-stage adhesion-improving layer 122d'. The polymer films 122c are laminated by thermocompression, for example, at a temperature of 295°C and a pressure of 4 MPa to 8 MPa for about 30 minutes. However, this feature according to the present disclosure is not specifically limited, since the lamination may be performed by a variety of known methods, including thermocompression.

Since the electronic component 122e (see FIG. 7e) is not yet mounted on the circuit board 122a in this step, there is no danger that the electronic component 122e may be damaged by the high-temperature and high-pressure lamination process performed for a long time.

As illustrated in FIG. 7d, portions of the intermediate-stage adhesion-improving layer 122d', corresponding to the mounting section 122b and the terminal section 123 of the circuit board 122a, are removed, thereby completing the final adhesion-improving layer 122d.

Although not shown, the lamination process may be performed after portions corresponding to the mounting section 122b and the terminal section 123 of the circuit board 122a are removed from the polymer films 122c, which has not yet been subjected to lamination. It should be understood that this configuration shall also belong the equivalent range of the invention defined by the Claims.

The portions corresponding to the mounting section 122b and the terminal section 123 of the circuit board 122a may be removed by a variety of methods, such as laser engraving, and this feature according to the present disclosure is not specifically limited.

As illustrated in FIG. 7e, the functioning section 122 is completed by mounting the electronic component 122e on the mounting section 122b of the circuit board 122a.

Since the above-described adhesion-improving layer 122d is additionally provided on the functioning section 122, the adhesion of the encapsulating section 124 to the functioning section 122 is increased. That is, when the surface of the circuit board 122a is made of a different material from the polymer of the encapsulating section 124 or, in the case that the surface is made of a polymer material the same as the polymer of the encapsulating section 124, has a high melting temperature equal to the melting temperature of the polymer of the encapsulating section 124, the adhesion-improving layer 122d made of a lower melting temperature may be additionally provided on an outer portion of the functioning section 122. The adhesion-improving layer 122d may be injected in a liquid state to be cured later, thereby further increasing the adhesion between the polymer of the encapsulating section 124 and the functioning section 122,

Although the a bio-implantable device and the method of manufacturing the same according to the present disclosure have been described with respect to the embodiments thereof, the scope of the present invention to be protected is not limited to specific embodiments, and those skilled in the art will appreciate that various substitutions, modifications, and alterations are possible without departing from the scope of the present disclosure. The foregoing embodiments disclosed herein and the accompanying drawings are provided for illustrative purposes rather than limiting the principle of the present invention. The scope of the principle of the present invention is not limited by the foregoing embodiments or the accompanying drawings. It should be understood that the scope of the present invention shall be defined by the appended Claims and all of their equivalents fall within the scope of the present invention.

## Claims

1. A bio-implantable device comprising:
one or more microwire arrays respectively comprising:
a substrate section made of a polymer;
a wire section provided on one surface of the substrate section; and
a cover section made of a polymer, the cover section being adhered to the one surface of the substrate section on which the wire section is provided to protect the wire section and expose at least one end of the wire section, thereby providing a pad section; and
one or more packages respectively connected to one end of a corresponding microwire array among the microwire arrays, and respectively comprising:
a functioning section electrically connected to the pad section via a terminal section; and
an encapsulating section made of a polymer, the encapsulating section being in contact with the functioning section and the pad section without gaps to protect the functioning section and the pad section, and extending to and being adhered to portions of the substrate section and the cover section adjacent to the pad section,
wherein the encapsulating section is provided by curing of the polymer, an initial state of which is liquid.

2. The bio-implantable device according to claim 1, wherein the functioning section comprises a adhesion-improving layer wrapping portions of the circuit board, except for an electronic component mounted on the circuit board, a mounting section of the circuit board allowing the electronic component to be mounted thereon, and a terminal section electrically connected to the pad section, wherein a melting temperature of the adhesion-improving layer is lower than a melting temperature of the polymer of the encapsulating section.

3. The bio-implantable device according to claim 1 or 2, wherein a melting temperature of the encapsulating section is higher than a melting temperature of either the substrate section or the cover section.

4. A method of manufacturing a bio-implantable device, comprising:
(a) a step of preparing one or more microwire arrays respectively comprising a wire section disposed between a substrate section and a cover section made of polymers, with a portion of the wire section being exposed from the cover section to provide a pad section on at least one end thereof;
(b) a step of electrically connecting the one or more microwire arrays and one or more functioning sections by electrically connecting terminal sections of the one or more functioning sections to the pad sections of the one or more microwire arrays; and
(c) a step of providing an encapsulating section in contact with the one or more functioning sections and the pad sections without gaps to protect the one or more functioning sections and the pad sections by placing the electrically-connected one or more microwire arrays and functioning sections in a mold, injecting a liquid polymer into the mold, and cooling the injected polymer, the encapsulating section extending and being adhered to portions of the substrate sections and the cover sections adjacent to the pad sections.

5. The method according to claim 4, wherein the functioning section comprises a adhesion-improving layer wrapping portions of the circuit board, except for an electronic component mounted on the circuit board, a mounting section of the circuit board allowing the electronic component to be mounted thereon, and a terminal section, wherein a melting temperature of the adhesion-improving layer is lower than a melting temperature of the polymer of the encapsulating section.

6. The method according to claim 5, wherein the adhesion-improving layer is prepared by a process comprising:
a step of wrapping the circuit boards with a polymer film by laminating the polymer film; and
a step of removing portions of the laminated polymer film corresponding to the mounting sections of the circuit boards and the terminal sections.

7. The method according to any one of claims 4 to 6, wherein each of the one or more microwire arrays is prepared by the step (a) comprising:
(a1) a step of providing the wire section on one surface of the substrate section made of a polymer;
(a2) a step of attaching the cover section made of the polymer to the one surface of the substrate section on which the wire section is provided; and
(a3) a step of providing the pad section by processing one end of the cover section.

8. The method according to any one of claims 4 to 6, further comprising:
(a4) a step of providing an alignment hole in regions in which the wire section is not present to penetrate the substrate section and the cover section before or after the step (a3).

9. The method according to any one of claims 4 to 6, wherein the electrical connection of the one or more microwire arrays and one or more functioning sections (b) is performed by bonding the terminal sections and the pad sections using silver epoxy or hot lead.

10. The method according to any one of claims 4 to 6, wherein the step (c) comprises:
(c1) a step of placing the electrically-connected one or more microwire arrays and functioning sections in a first mold;
(c2) a step of injecting a liquid polymer into the first mold and cooling the injected polymer to provide an upper encapsulating section in contact with upper portions of the one or more functioning sections and the pad sections without gaps to protect the one or more functioning sections and the pad sections, the upper encapsulating section extending and being adhered to portions of the substrate sections adjacent to the pad sections;
(c3) a step of placing the electrically-connected one or more microwire arrays and functioning sections, on which the first encapsulating section is provided, in a second mold; and
(c4) a step of providing a lower encapsulating section in contact with lower portions of the one or more functioning sections and the pad sections without gaps to protect the one or more functioning sections and the pad sections by injecting a liquid polymer into the second mold and cooling the injected polymer, the lower encapsulating section extending and being adhered to portions of the substrate sections adjacent to the pad sections.

11. The method according to any one of claims 4 to 6, further comprising:
(d) a step of additionally providing one or more encapsulating sections by adding one or more functioning sections via the microwire array in a state in which one or more encapsulating sections are provided, placing resultant structures in a mold, injecting a liquid polymer into the mold, and cooling the injected polymer, the one or more encapsulating sections being in contact with the added one or more functioning sections and the connected pad sections without gaps to protect the added one or more functioning sections and the connected pad sections, and extending and being adhered to portions of the substrate sections of the intermediating microwire array and the cover sections adjacent to the pad sections.
